# EUROPEAN PATENT APPLICATION

(11) **EP 4 016 078 A1**
(43) Date of publication of application: **22.06.2022**
(21) Application number: 20215280.7
(22) Date of filing: 18.12.2020
(51) Int. Cl.: G01N 33/50, G01N 33/68

(54) **METHOD USEFUL IN TOLERANCE INDUCTION THERAPY AND KITS THEREFORE**

(71) Applicant: Rheinische Friedrich-Wilhelms-Universität Bonn, 53113 Bonn (DE)
(72) Inventor: KURTS, Christian, Prof. Dr., 53127 Bonn (DE); MEIßNER, Mirjam, 53121 Bonn (DE); OLDENBURG, Johannes, Prof. Dr., 53177 Bonn (DE); ALBERT, Thilo, Dr., 53347 Alfter (DE); GOTOT, Janine, Dr., 53842 Troisdorf (DE)
(74) Representative: Gramm, Lins & Partner Patent- und Rechtsanwälte PartGmbB

(57) **Abstract**

The present invention relates in a first aspect to a method for the stratification of a therapeutic regimen of a subject afflicted or suspected to be afflicted with an immune regulatory antibody-mediated disease based on the immune status of said subject, the method is based on determining the level or amount of expression of PD-1 in a predetermined subset of B-cells, thus, reflecting the immune tolerance status against an immune tolerance inducing compound of said subject. In addition, a method for monitoring of the development or progress of a treatment in a subject based on an administration of immune-tolerance-inducing compound containing antigenic epitopes recognized by the B-cells producing these antibodies. Further, a method for determining the risk of developing antibody-producing B-cells based failure of immune tolerance induction (ITI) treatment in a subject is provided. Moreover, the use of PD-1 expression as a marker in antibody-mediated disease based on B-cell tolerance status is described. Finally, a kit for use in determining antibody-producing B-cells for determining B-cell tolerance status in a predetermined set of B-cells is described.

## Description

The present invention relates in a first aspect to a method for the stratification of a therapeutic regimen of a subject afflicted or suspected to be afflicted with an immune regulatory antibody-mediated disease based on the immune status of said subject, the method is based on determining the level or amount of expression of PD-1 in a predetermined subset of B-cells, thus, reflecting the immune tolerance status against an immune tolerance inducing compound of said subject. In addition, a method for monitoring the development or progress of a treatment in a subject based on an administration of immune-tolerance-inducing compounds containing antigenic epitopes recognized by the B-cells producing these antibodies. Further, a method for determining the risk of developing antibody-producing B-cells based failure of immune tolerance induction (ITI) treatment in a subject is provided. Moreover, the use of PD-1 expression as a marker in antibody-mediated disease based on B-cell tolerance status is described. Finally, a kit for use in determining antibody-producing B-cells for determining B-cell tolerance status in a predetermined set of B-cells is described.

### Prior Art

Immunologic tolerance is a state of immune unresponsiveness specific to a particular antigen or set of antigens induced by previous exposure by that antigen or set of antigens. Tolerance is generally accepted to be an active process, in essence, a learning experience for immune cells, like B-cells and T-cells. For example, immune-tolerance-induction (ITI) treatment is conducted to eradicate inhibitors of therapeutic active compounds.

Haemophilia is a rare inherited bleeding disorder due to the deficiency of Factor VIIII (FVIII, haemophilia A) or Factor IX (FIX, haemophilia B) in plasma. Haemophilia A is an X-chromosome-linked-inherited bleeding disorder with an incidence of 1 in 5000 male births. Resulting from limitations within the Factor VIII (FVIII) gene, the pathophysiological characteristics of haemophilia A show low plasma level or activity of Factor VIII protein. Patients with haemophilia A suffer from lifelong bleeding tendencies, such as spontaneous or traumatic bleeding episodes. At present, standard treatment includes a protein replacement therapy with plasma-derived or recombinant Factor VIII, which is typically administered by intravenous infusion. That is, replacement therapy is a cornerstone of haemophilia management since it allows to control active bleeding by on-demand episodic treatment and/or to prevent recurrent bleeds by regular prophylaxis.

However, the development of neutralizing alloantibodies directed against Factor VIII or Factor IX (also referred to as "inhibitors"), is a main complication of haemophilia treatment because it renders bleeding control difficult and standard prophylaxis unfeasible. That is, this main complication of replacement therapy in haemophilia is the formation of anti-Factor VIII inhibitory antibodies, the inhibitors, that occurs in approximately 30 % of all patients.

Inhibitor development is more common among patients with haemophilia A than in those with haemophilia B as well as in patients with severe haemophilia than in those with mild haemophilia. The risk of developing inhibitors is maximized after the first 10 to 15 exposure days to the antigen, namely, the Factor VIII or Factor IX concentrates, hence inhibitors occur mostly in children with severe haemophilia A. Among these inhibitors almost one third are transitioned and spontaneously disappear without sequel and the need for specific treatment regimens. In the presence of persistent high-affinity inhibitors, standard replacement therapy are no longer effective and recurrent joint bleeds are managed by on-demand treatment with bypassing agents. However, these bypassing agents, like recombinant activated Factor VII, recombinant Factor Vila, and activated prothrombinic complex concentrate, the haemostatic efficiency is suboptimal compared with Factor VIII replacement therapy. That is, high-affinity inhibitors neutralize the infused Factor VIII protein in the case of haemophilia A leading to increased morbidity and mortality.

Recently, a bispecific antibody, mimicking Factor VIII has been approved for prophylaxis in haemophilia A patients with inhibitors.

Therefore, the mainstay approach is to induce Factor VIII specific tolerance, the so-called immune tolerance induction therapy (ITI). Treatment of inhibitors is very expensive and represents an extreme burden to the patients and their families.

This ITI treatment generally consists of the regular administration of respective Factors, Factor VIII or Factor IX, to render the immune system tolerant to the antigen by preventing further production of the antibodies directed against Factor VIII and Factor IX, respectively. It is a demanding therapeutic regimen since it implies frequent intravenous injections, typically daily or every other day, in a subject with poor venous accesses as children and for a rather prolonged time. Namely, ITI involves repetitive injections of high doses of Factor VIII or Factor IX, respectively, for a long period of usually 1 to 3 years inducing the immunological phenomenon of "high zone tolerance". One of the most popular and most successful protocols for ITI is the "Bonn Protocol", which represents an entirely empirically developed protocol ( Brackmann HH, Oldenburg J, Schwaab R., Vox Sang 1996, 70: 30-5). Nevertheless, approximately 20 to 40 % of patients undergoing such ITI treatment do not achieve long-lasting peripheral tolerance.

The mechanism underlying the high zone immune tolerance in general, and ITI in particular, are unknown hampering the optimization of ITI accordingly. At present, no clinical assay to monitor ITI in patients is available. ITI optimization is a priority for haemophilia treatments and the replication of predictors of responses supporting or to offer ITI in patients who may benefit the most from it and to tailor it properly, thus, avoiding a waste of resources.

Success rates of ITI in haemophilia A has been defined by clinical and laboratory features, see e. g. Mariani G., et al., Semin. Thromb. Hemost., 29 (1), 69 to 79 (2003). Further steps in defining success and failure were developed, wherein a partial response is regarded as being a response with undetectable inhibitor titers but persistently abnormal Factor VIII recovery or half-life of 33 months of ITI in association with clinical response to Factor VIII replacement therapy without an anesthetic increase of the inhibitor titers. Inhibitor relapse was defined as inhibitor occurrence during the 12 month follow-up period on prophylaxis after success as evidenced by recurrent positive inhibitors titers or impaired Factor VIII pharmacokinetics.

Inhibitor titers at various time points prior to ITI have been confirmed as independent predictors of ITI outcome. However, inhibitor titer is not sufficient to monitor ITI in patients.

Already 40 years ago, high zone immune tolerance has been suspected to be mediated by suppressor T-cells in connection with macrophage responses. The existence of these types of suppressor T-cells was described 15 years later, namely, the so-called "regulatory T-cells" or "Tregs". It is known that induction of Tregs or transfer of Tregs suppress inhibitor formation in mice, although the underlying mechanism remains unclear. It is known that Tregs can suppress alloantibody production and it was previously reported that Tregs suppress autoreactive B-cells in an antigen-specific and contact-dependent manner via the programmed Death-1 signal pathway. The programmed Death-1 molecule is also known as PD-1 or CD279.

PD-1 is an activation-induced member of the extended CD28-CTLA-4 family described as being involved in suppressing T-cells. The molecule has been associated with T-cell exhaustion in chronic viral infection and allows tumors to escape CTL surveillance. Further, PD-1 blocking antibodies have emerged as extremely potent anticancer drugs. It is known from the clinic that patients treated with PD-1 develop auto-antibodies. However, the role of PD-1 in the formation of alloantibodies is unclear. PD-1 is expressed on T-cells and pro-B-cells. PD-1 binds to two ligands, namely, the PD-L1 and PD-L2 ligands. While the PD-L1 protein is upregulated on macrophage and dendritic cells as well as on T-cells and B-cells upon TCR and BCR signaling, PD-L2 expression is more restricted and is expressed mainly by dendritic cells and some tumor cell lines. PD-L1 is also known as CD274 or B7-homolog-1.

As noted, it is important to allow monitoring ITI in patients to predict ITI success, however, the definition of ITI outcome and predicted ITI outcome is required.

### Brief description of the present invention

The aim of the present invention is to provide a method and means allowing to monitor the ITI therapy in individuals and, generally, methods and means for the stratification of the therapeutic regimen, for monitoring in a subject afflicted with an immune regulatory antibody-mediated disease, the development or progress of a treatment based on an administration of an immune tolerance inducing compound. In addition, an aim of the present invention is to allow monitoring success of ITI treatment in a subject in need thereof.

In a first aspect, the present invention relates to a method for the stratification of the therapeutic regimen of a subject afflicted or suspected to be afflicted with an immune antibody-mediated disease based on the immune status of said subject including the steps of
a) providing a sample containing B-cells from said subject;
b) determining the level or amount of the expression of PD-1 in a predetermined subset of B-cells present in said subject reflecting the immune status of said subject;
c) determining the therapeutic regimen of said subject based on the level or amount of the expression of PD-1 in a predetermined subset of B-cells said B-cells reflect the immune status of said subject with respect to said disease.

Moreover, the present invention relates in a further embodiment to a method for monitoring in a subject afflicted with an immune regulatory antibody-mediated disease, the development or progress of a treatment based on an administration of an immune-tolerance-inducing compound containing antigenic epitopes recognized by the B-cells producing these antibodies to said subject during therapy, including the steps of
a) determining the level or amount of the expression of PD-1 in a predetermined subset of B-cells present in a sample obtained from said subject at a first time point and,
b) optionally, determining the level or amount of the expression of PD-1 in a predetermined subset of B-cells present in a sample obtained from said subject at a second time point; and
c) comparing the level or amount of the expression of PD-1 by said B-cells determined in step a) to the level or amount detected in step b) or to a reference value.

In another embodiment, the present invention relates to a method for determining the risk of developing antibody-producing B-cells based failure of immune tolerance induction (ITI) treatment in a subject undergoing said treatment to ITI comprising the steps of
a.) determining the level or amount of the expression of PD-1 in a predetermined subset of B-cells being specific against the immune tolerance inducing compound present in said sample at a first time point, said predetermined subset of B-cells being specific against the immune tolerance inducing compound;
b.) optionally determining the level or amount of the expression of PD-1 in a predetermined subset of B-cells being specific against the immune tolerance inducing compound present in a sample obtained from said subject at a second time point;
c.) determining the risk of failure of ITI therapy in said subject based on the level or amount of the expression of PD-1 in the predetermined subset of B-cells being specific against the immune tolerance inducing compound.

The present inventors recognized that the expression level of PD-1 by B-cells specific for the immune tolerance inducing compound, namely, in the subset of B-cells reflecting the immune tolerance status of the subject against the immune tolerance inducing compound is indicative for success of treatment, in particular, success of ITI therapy in e. g. treating of haemophilia A. Generally, component replacement therapy and success thereof can be monitored based on the PD-1 expression, in particular, when inhibitors develop which are eradicated by immune tolerance induction therapy.

In a further aspect, the present invention relates to the use of PD-1 as a marker for the stratification of the therapeutic regimen of a subject afflicted or suspected to be afflicted with an antibody-mediated disease based on the B-cell tolerance status of the subject. The use is particularly useful in determining success of ITI in the treatment of subject in need thereof, in particular, in subjects afflicted with haemophilia A.

Finally, a kit for use in determining the antibody producing B-cells by flow cytometry is provided. Said kit comprises antibodies to differentiate immune cells as well as an antibody against PD-1. Moreover, said kit contains a labelled allogenic compound, typically the allogenic compound is the immune-tolerance-inducing compound administered to a subject.

Further, methods of treatment are provided including the step of administering to a subject being afflicted with an immune antibody mediated disease, like subjects undergoing ITI, a therapeutically effective amount of an agonist of PD-1, like recombinant PD-L1 are an agonistic antibody against the PD-1 molecule expressed on B-cells. In case of ITI, administration of the agonist of PD-1 may be conducted simultantaneously, separately or sequentially with the administration of the immune-tolerance-inducing compound. Particularly, during the initial injections of the administered allogen, the agonist of PD-1 is administered.

### Brief description of the figures

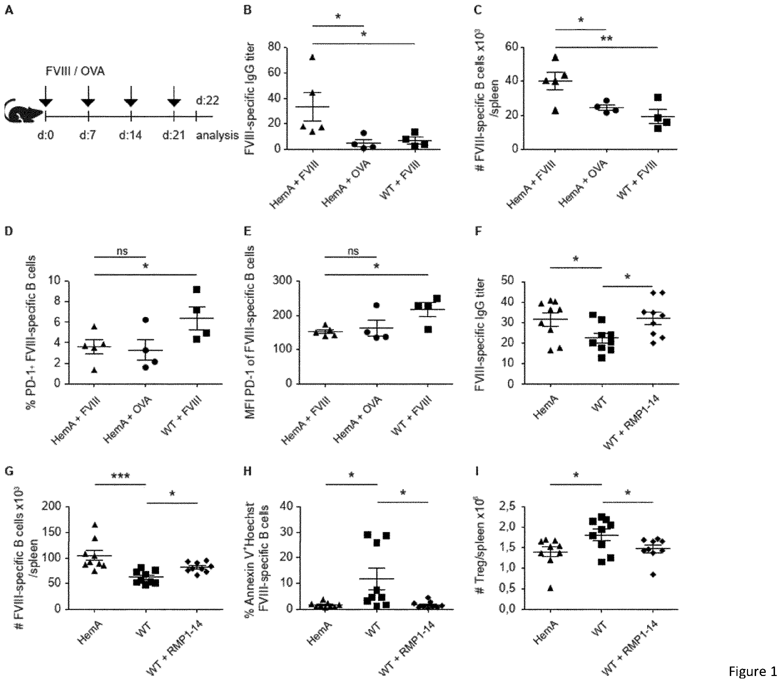

**Figure 1****: PD-1 suppresses inhibitor formation in vivo**
   **a,** Experimental scheme. A scaled standard dose (80 IU/kg) of human FVIII was intravenously injected into HemA (triangle) and WT (square) in weekly intervals. As a control 10 µg of the foreign antigen OVA was also applied to HemA mice (circle) in weekly intervals. One day after the last injection splenocytes and blood serum were harvested and further analyzed by flow cytometry and ELISA, respectively.
   **b,** ELISA-based quantification of the FVIII-specific IgG antibody titer in the serum of HemA and WT mice.
   **c,** Quantification of the number of FVIII-specific B cells in the spleen of HemA and WT mice after treatment with FVIII or OVA by flow cytometry.
   **d, e,** The expression of PD-1 on FVIII-specific B cells is presented as percentage of FVIIIspecific B cells that express PD-1 (d) as well as MFI of PD-1 on FVIII-specific B cells (e).
   **f-i,** PD-1 inhibition in WT mice by the intraperitoneal injection of an inhibitory antibody that was applied twice a week.
   **f,** ELISA-based quantification of the FVIII-specific IgG antibody titer in the serum of HemA (triangle) and WT (square) mice and WT mice that were additionally treated with aPD-1 (upright square).
   **g,** Quantification of the number of FVIII-specific B cells by flow cytometry. **h,** Early apoptotic cells are presented as percentage of Annexin V+ and Hoechst-FVIII-specific B cells after *in vitro* re-stimulation with 0.25 µg FVIII overnight.
   **i,** Number of CD4+Foxp3+ regulatory T cells in the spleen of treated mice. Data are presented as mean +/- SEM. *P <0.05; **P < 0.01; ***P < 0.001 (ANOVA and Bonferroni).
**Figure 2****: The anti-FVIII immune response in hemophilic mice is antigen-specific and restricted to PD-1**

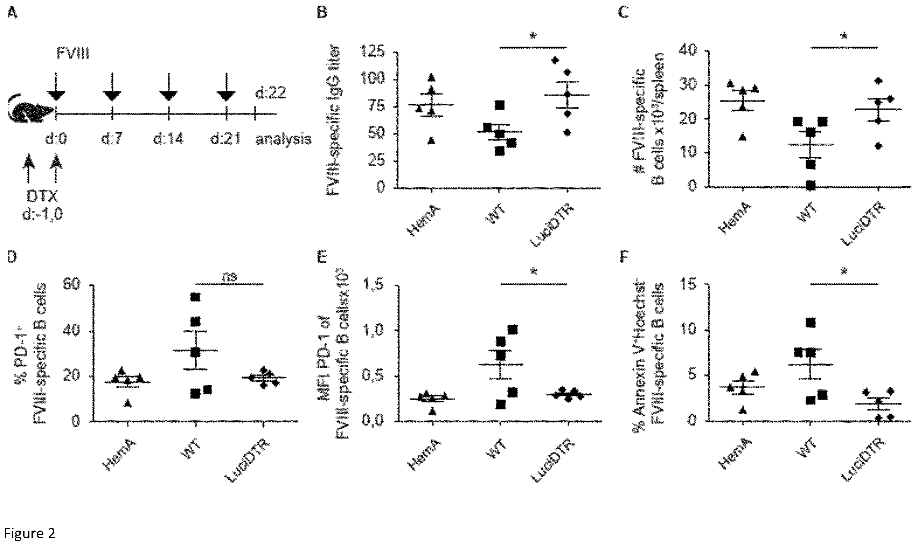

   **Fig. 2a****,** Gating strategy for FVIII-specific B cells of mice treated with FVIII or OVA. **b-j** A scaled standard human dose (80 IU/kg) of FVIII was intravenously injected into HemA (triangle) and WT (square) in weekly intervals. As control 10 µg of the foreign antigen OVA was also applied to HemA mice (circle) in weekly intervals. (**b**) Number of antibody-forming units represents the quantification of FVIII-specific B cells in 10⁷ splenocytes. Percentage of FVIII-specific B cells expressing Fas (**c**) CD80 (**e**) or CD86 (**g**). MFI of expressing Fas (**d**) CD80 (**f**) or CD86 (**h**) on FVIII-specific B cells **i, j,** PD-1 blockage in WT mice by the intraperitoneal injection of an anti-PD-1 inhibitory antibody that was applied twice a week (upright square). Percentage of PD-1 expressing FVIII-specific B cells (**i**) and the MFI of PD-1 on FVIII-specific B cells (**j**).
**Figure 3****: Inhibition of FVIII-specific B cells is mediated by Foxp3⁺ Tregs**
   **a,** Experimental scheme. HemA (triangle), WT (square), and LuciFoxp3DTR (upright square) mice were intravenously injected with a scaled standard human dose (80 IU/kg) of FVIII in weekly intervals. Foxp3+ Tregs were depleted in LuciFoxp3DTR mice by injecting 15 ng/g mouse DTX intraperitoneally at day -1 and 0 of the experiment. One day after the last injection of FVIII splenocytes and blood serum were harvested and further analyzed by flow cytometry and ELISA, respectively.
   **b,** ELISA-based quantification of the FVIII-specific IgG antibody titer in the serum of HemA and WT mice.
   **c,** Quantification of the number of FVIII-specific B cells in the spleen of HemA and WT mice after treatment with FVIII by flow cytometry.
   **d, e,** Percentage of PD-1 expressing FVIII-specific B cells (d) and the MFI of PD-1 on FVIIIspecific B cells (**e**).
   **f,** Early apoptotic cells are presented as percentage of Annexin V⁺ and Hoechst-FVIII-specific B cells after in vitro re-stimulation with 0.25 µg FVIII overnight. Data are presented as mean +/- SEM. *P <0.05; **P < 0.01; ***P < 0.001 (ANOVA and Bonferroni).

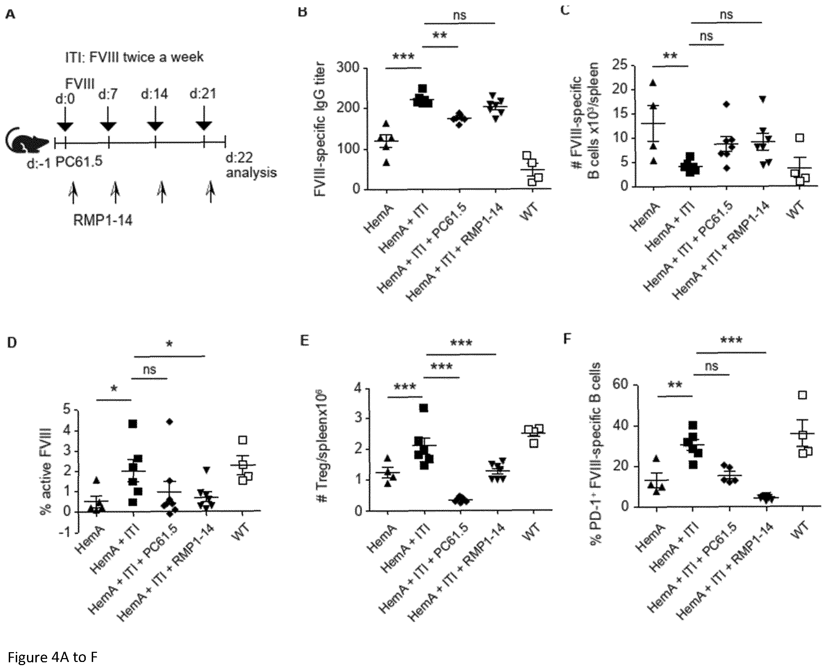

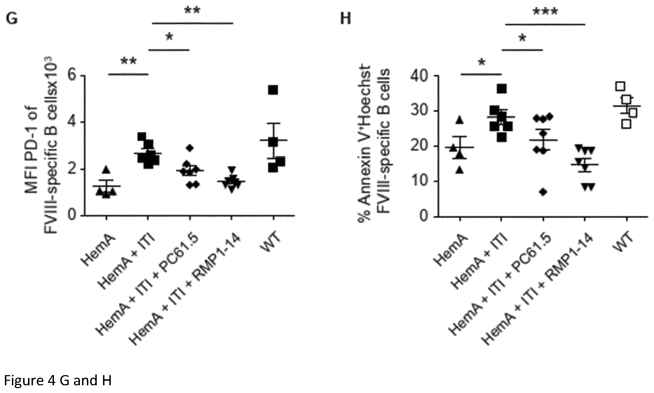

**Figure 4****: PD-L1 on Tregs abrogates inhibitor formation in hemophilic mice.**
   **a,** Experimental setup for the adoptive transfer of regulatory T cells and the subsequent treatment of mice with FVIII. Tregs were isolated either from WT or PD-L1-/- mice and 1x106 cells were transferred into HemA mice by intravenous injections. Starting from the next day, HemA (triangle), WT (square), and HemA mice that received Tregs from WT (circle) or PD-L1-/- (triangle point down) mice were intravenously injected with a scaled standard human dose (80 IU/kg) of FVIII in weekly intervals. One day after the last injection splenocytes and blood serum were harvested and further analyzed by flow cytometry and ELISA, respectively.
   **b,** Titer of FVIII-specific IgG antibodies in the serum of FVIII-treated mice. **c,** Quantification of the number of FVIII-specific B cells in the spleen of HemA and WT mice after treatment with FVIII by flow cytometry
   **d, e,** Percentage of PD-1 expressing FVIII-specific B cells (d) and the MFI of PD-1 on FVIIIspecific B cells (**e**).
   **f,** Early apoptotic cells are presented as percentage of Annexin V+ and Hoechst-FVIIIspecific B cells after in vitro re-stimulation with 0.25 µg FVIII overnight. Data are presented as mean +/- SEM. *P <0.05; **P < 0.01; ***P < 0.001 (ANOVA and Bonferroni).

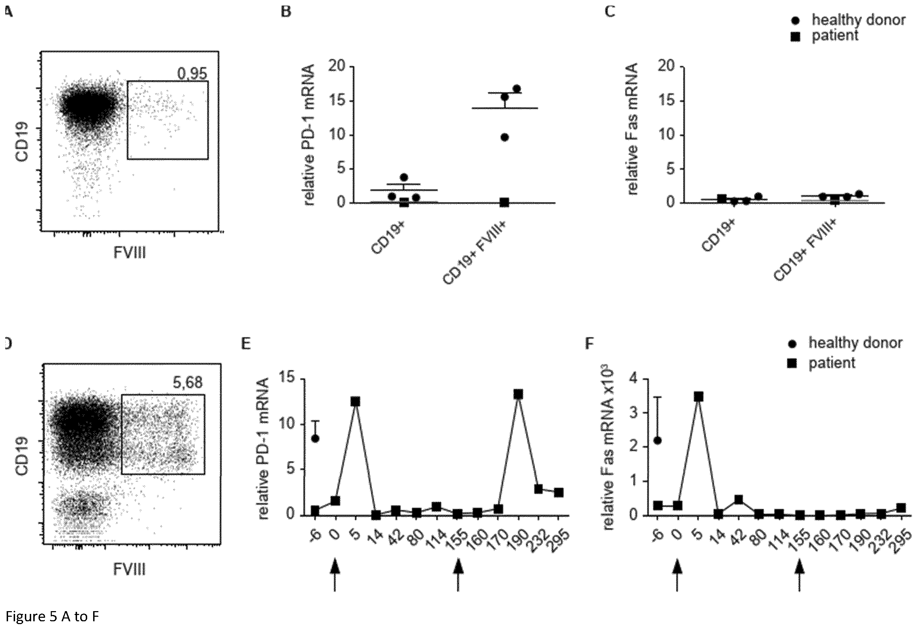

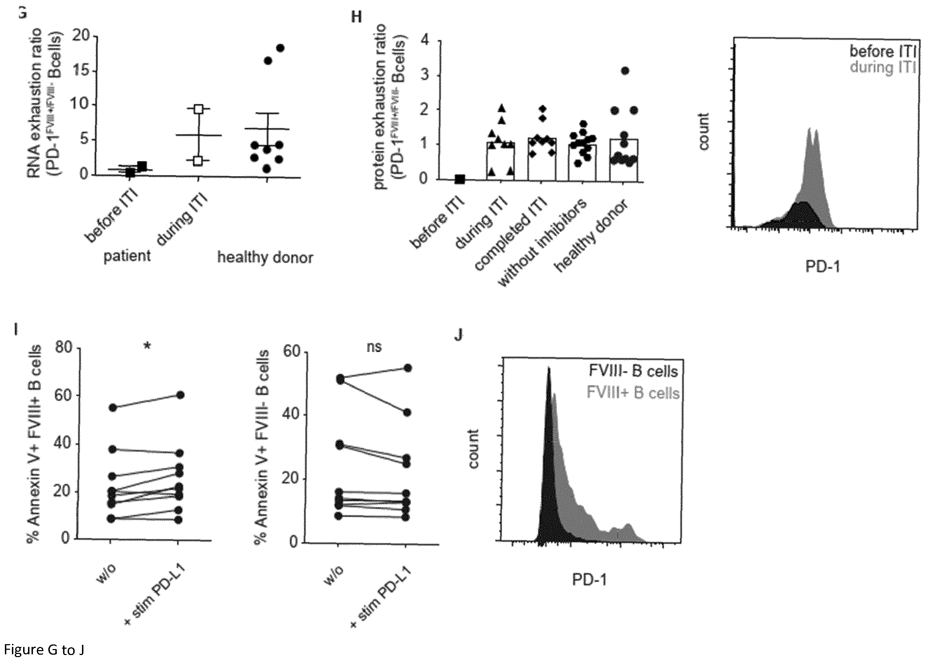

**Figure 5****: ITI restores PD-1 expression leading to increased apoptosis of FVIII-specific B cells**
   **a,** Experimental scheme. A scaled standard human dose (80 IU/kg) of FVIII was intravenously injected into HemA (triangle) and WT (square) in weekly intervals. Immune tolerance induction in HemA mice (filled square) was achieved by injecting of FVIII twice a week. CD25+ Tregs were depleted in ITI receiving HemA mice (upright square) by the intraperitoneal injection of 250 µg depleting aCD25 antibody (PC61.5) one day prior each FVIII injection. The PD-1 axis was inhibited in HemA mice (triangle point down) by injecting an aPD-1 inhibitory antibody (RMP1-14) intraperitoneally 3 h after each FVIII treatment. One day after the last injection of FVIII splenocytes and blood serum were harvested and further analyzed by flow cytometry and ELISA, respectively.
   **b,** Titer of FVIII-specific IgG antibodies in the serum of FVIII-treated mice.
   **c,** Quantification of the number of FVIII-specific B cells in the spleen of HemA and WT mice after treatment with FVIII by flow cytometry.
   **d,** Percentage of active human FVIII protein in the serum of treated mice.
   **e,** Number of Tregs in the spleen of treated mice.
   **f, g,** Percentage of PD-1 expressing FVIII-specific B cells (f) and the MFI of PD-1 on FVIII-specific B cells (g).
   **h,** Early apoptotic cells are presented as percentage of Annexin V+ and Hoechst-FVIII-specific B cells after *in vitro* re-stimulation with 0.25 µg FVIII overnight.
      Data are presented as mean +/- SEM. *P <0.05; **P < 0.01; ***P < 0.001 (ANOVA and Bonferroni).

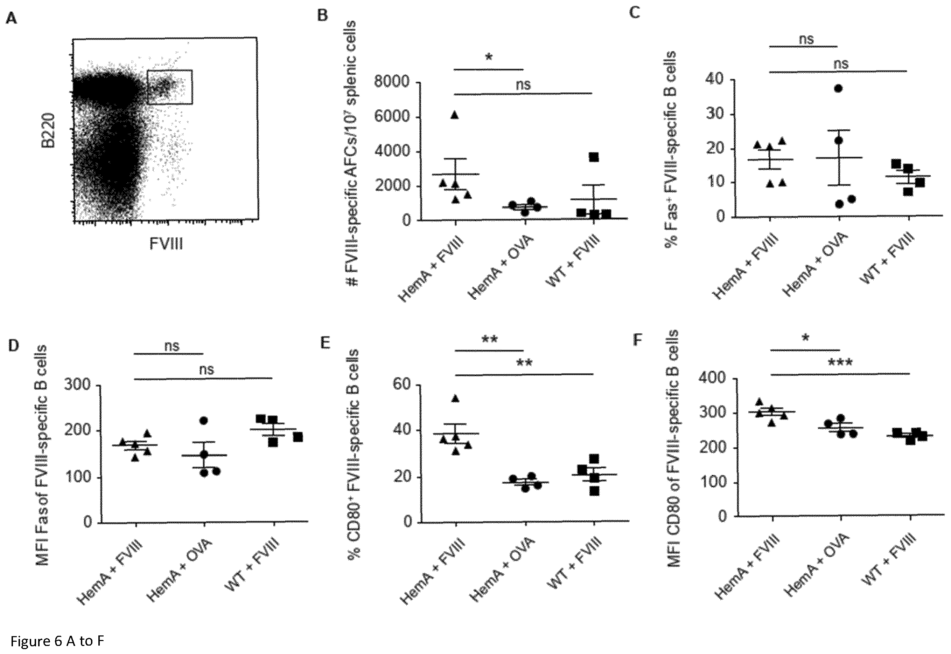

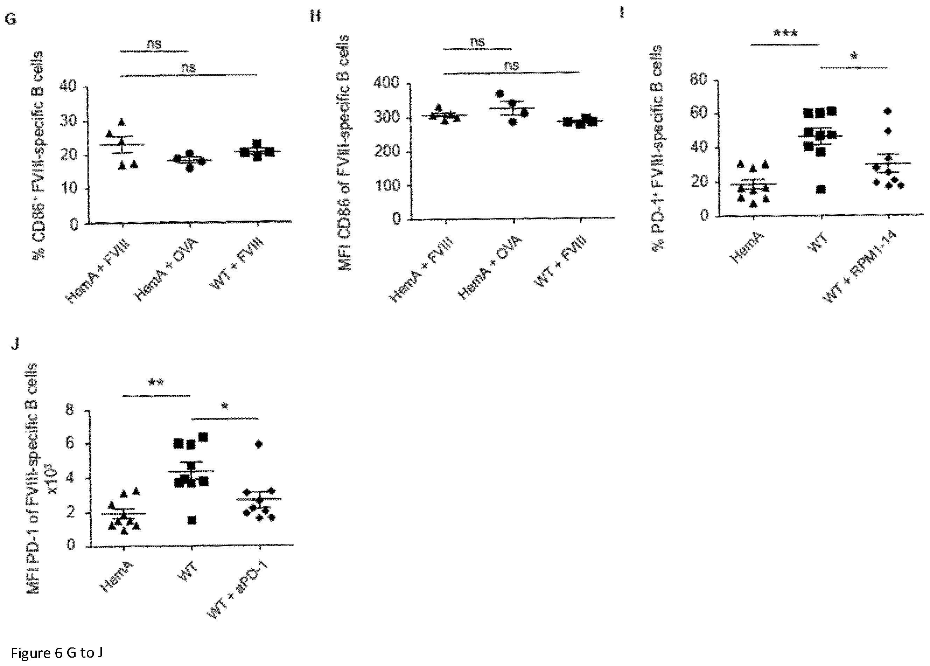

**Figure 6****: Accumulation of induced Tregs during ITI in hemophilic mice.**
   A scaled standard human dose (80 IU/kg) of FVIII was intravenously injected into HemA (triangle) and WT (square) in weekly intervals. Immune tolerance induction in HemA mice (filled square) was achieved by injecting FVIII twice a week. CD25+ Tregs were depleted in ITI receiving HemA mice (upright square) by the intraperitoneal injection 250 µg depleting aCD25 antibody (PC61.5) one day prior each FVIII injection. The PD-1 axis was inhibited in HemA mice (triangle point down) by injecting an aPD-1 inhibitory antibody (RMP1-14) intraperitoneally 3 h after each FVIII treatment. One day after the last injection of FVIII splenocytes were obtained for analysis by flow cytometry. Percentage of Neuropilin-1- and Helios- Foxp3+ CD4+ T cells.

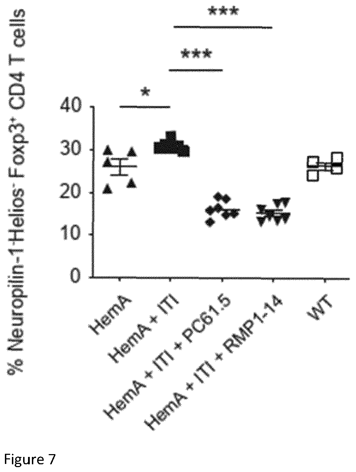

**Figure 7****: PD-1 expression on human FVIII-specific B cells renders them sensitive for PD-L1 mediated suppression**
   **a,** Gating strategy for sorting FVIII-specific B cells of human blood samples. CD19+ B cells were gated and sorted for their ability to bind to fluorescently labeled FVIII protein. mRNA was extracted from sorted FVIII-specific B cells and analyzed by RT-PCR.
   **b, c,** Relative mRNA expression of PD-1 (b) and Fas (c) in FVIII-specific B cells of Haemophilia A patients with inhibitors (square) or healthy donors (circle).
   **D,** Gating strategy for sorting FVIII-specific B cells of human blood samples from a Haemophilia A patient during a cycle of ITI (square) or healthy donors (circle). mRNA was extracted from sorted FVIII-specific B cells and analyzed by RT-PCR at different time points during ITI.
   **e, f,** Relative mRNA expression of PD-1 (e) and Fas (f) in FVIII-specific B cells during ITI.
   **g,** RNA exhaustion ratio of PD-1 in FVIII-specific B cells and antigen-unspecific B cells in a Haemophilia A patient before ITI (filled quare), during ITI (square), and in healthy control donors (circle).
   **h,** Protein exhaustion ratio of PD-1 on FVIII-specific B cells and antigen-unspecific B cells in Haemophilia A patients before ITI (square), during ITI that received a FVIII injection <24h before analysis (triangle), with a completed ITI (upright square), without inhibitor titers (hexagon) and healthy control donors (circle). The MFI of PD-1 on FVIII-specific B cells before (black) and during ITI (grey) is presented as histogram.
   **i,** Percentage of apoptotic Annexin V+ FVIII-specific B cells and antigen-unspecific B cells without and with stimulation with a stimulating aPD-L1 antibody in vitro. j, PD-1 expression on FVIII-specific and antigen-unspecific B cells of healthy control donors after stimulation with a stimulating aPD-L1 antibody in vitro. Data are presented as mean +/- SEM. *P <0.05; **P < 0.01; ***P < 0.001 (paired Student's t test).

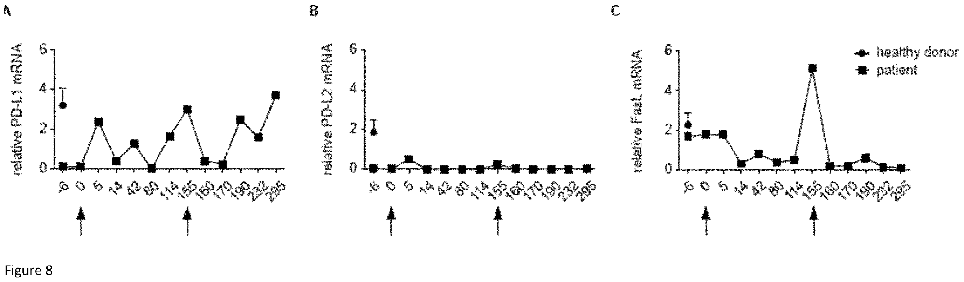

**Figure 8****: PD-1 is the operating molecule mediating tolerance towards FVIII in humans.**
   **a, b, c,** mRNA was extracted from sorted FVIII-specific B cells and analyzed by RT-PCR at different time points during ITI. Relative mRNA expression of PD-L1 (**a**), PD-L2 (**b**), and FasL (**c**) in FVIII-specific B cells of a Hemophilia A patient during ITI (square) or healthy donors (circle). Data are presented as mean +/- SEM.

### Detailed description of the present invention

In a first aspect, the present invention relates to a method for the stratification of the therapeutic regimen of a subject afflicted or suspected to be afflicted with an immune antibody-mediated disease based on the immune status of said subject including the steps of
a) providing a sample containing B-cells from said subject;
b) determining the level or amount of the expression of PD-1 in a predetermined subset of B-cells present in said subject, said predetermined B-cells reflect the immune status of said subject;
c) determining the therapeutic regimen of said subject based on the level or amount of the expression of PD-1 in a predetermined subset of B-cells reflecting the immune status of said subject with respect to said disease.

As used herein, the term "immune antibody-mediated disease" refers to a disease caused by antibodies against an infused recombinant antigen which is absent, reduced in expression or generated in truncated forms in the diseased patients for example due to mutations in the gene encoding the substituted protein.

Further, the term "based on the immune status" refers to the existence and immunological phenotype of reacting antigen-specific B cells.

The term "B-cells ... reflecting the immune status ..." refers to B-cells expressing on their surface antibodies directed to a compound to which the immune tolerance status is directed to, e. g. antibodies against FVIII.

The term "immune-tolerance-inducing-compound" refers to compounds which can initiate an antigen-specific tolerization based on its application.

As used herein, the term "comprising" or "comprises" or "containing" or "contains" includes the embodiment of "consist of" or "consisting of".

As used herein, the term "antigenic structure" or "antigenic epitope" refers to a structure present in an antigen capable of causing a cellular or hormonal immune response. The antigenic structure, also known as antigenic epitope or epitope in general, is part of the antigen which is presented by the MHC or MHC-like molecules. Further, the epitope or antigenic structure represent the part of an antigen recognized by antibodies directed against said antigen.

As used herein, the term "individual" or "subject" which is used herein interchangeably refers to a mammal, particularly preferred a human.

As used herein, the term "reference value" refers to an index value, a value derived from one or more computer indices, a value derived from a subject or a cohort of a subject of not afflicted subjects or a reference value obtained from individuals with successful treatment, in particular ITI or a subject or cohort of subject with failed ITI treatment, respectively.

Further, the term "determining" as used herein refers to assessing the presence, absence, quantity, level or amount of the mentioned compound within a clinical or subject derived sample. In particular, the term "determining" refers to asses physically the level or amount of the expression of PD-1 in a sample.

Determination can be effected on nucleic acid level as well as on protein level. That is, either expression (qualitatively or quantitatively) can be determined on miRNA basis or based on the expression of the protein on the surface of the respective B-cells.

The present invention is based on the finding that antibodies, like alloantibodies against an infused recombinant antigen, neutralizing the immune-tolerance-inducing-compounds are suppressed via PD-1 signalling and inducing tolerance re-enable the regulatory mechanism, thus, allowing ITI success. Namely, the present inventors succeeded in developing an assay to monitor ITI success based on PD-1 expression of B-cells producing antibodies against antigenic epitopes present on the immune-tolerance-inducing-compound.

In a further aspect, the present invention relates to a method for monitoring in a subject afflicted with an immune antibody-mediated disease the development or progress of a treatment based on an administration to said subject of an immune-tolerance-inducing compound containing antigenic epitopes recognized by the B-cells producing these antibodies during therapy, including the steps of
a) determining the level or amount of the expression of PD-1 in a predetermined subset of B-cells which are B-cells producing antibodies against the immune-tolerance-inducing compound present in a sample obtained from said subject at a first time point and,
b) optionally, determining the level or amount of the expression of PD-1 in said predetermined subset of B-cells which are B-cells producing antibodies against the immune-tolerance-inducing compound present in a sample obtained from said subject at a second time point; and
c) comparing the level or amount of the expression of PD-1 by said B-cells determined in step a) to the level or amount detected in step b)B or to a reference value.

Moreover, the present inventors aim to provide a method for determining the risk of developing antibody-producing B-cells based failure of immune tolerance induction (ITI) treatment in a subject undergoing said treatment to ITI by administering an immune-tolerance-inducing compound comprising the steps of
a.) determining the level or amount of the expression of PD-1 in a predetermined subset of B-cells being specific against the immune tolerance inducing compound present in said sample at a first time point;
b.) optionally determining the level or amount of the expression of PD-1 in said predetermined subset of B-cells being specific against the immune tolerance inducing compound present in a sample obtained from said subject at a second time point;
c.) determining the risk of failure of ITI therapy in said subject based on the level or amount of the expression of PD-1 in a predetermined subset of B-cells being specific against the immune tolerance inducing compound.

The expression level or amount of PD-1 expression on the antigen specific B-cells, namely, the B-cells producing antibodies directed against a specific antigen, e. g. the immune-tolerance-inducing-compound in ITI or other compounds administered to a subject by way of replacement therapy is important to establish B-cell tolerance against an antigen administered. That is, low or decreased level of amount of PD-1 expression on the antigen specific B-cells are indicative that immune tolerance is not induced or present against the specific antigen. For example, the low or decreased level can be determined by comparison with a different antigen specific B cell other than the B cells specific to the alloantigen, e.g. antigen specific B cells against a foreign antigen, like tetanus toxin or other vaccines. Alternatively, the level is compared to a different antigen administered or detected at the same time. Rather, the antigen is recognized and removed accordingly. Further, it is possible to detect different B-cells directed against a different antibody and determining the expression level or amount of PD-1 of said different subset of B-cells.

In contrast, it has been recognized by the inventors that the expression level or amount of other markers including activation markers of immune cells do not allow to identify B-cell tolerance induction in said subject.

In an embodiment, the method according to the present invention refers to a predetermined subset of B-cells being B-cells which produce antibodies directed against the B-cell tolerance-inducing-compound administered in ITI therapy. In another embodiment, the predetermined subset of B-cells are B-cells producing antibodies directed against compounds administered to a subject in need thereof, e. g. in case of replacement therapies. A typical example is the ITI therapy in case of haemophilia A or haemophilia B. Other replacement therapies are known, e. g. for Fabry disease or other diseases including Lysosomal storage diseases (Pompe's disease, Gaucher's disease and Fabry's disease) where the treatment includes a replacement therapy, in case of Fabry disease enzyme replacement therapy, whereby compounds or molecules which are decreased in function, are substituted by external administration of respective components. In case of haemophilia A Factor VIII is administered, in case of Fabry disease, the enzyme Agalsidase β is administered or alpha-Galactosidase.

The method according to the present invention requires a sample obtained from the subject to be analysed.

Typically, the sample is a sample containing B-cells. Said sample is obtained from blood, in particular, serum.

By known means, a predetermined subset of B-cells is identified. For example, the predetermined subset of B-cells are human B-cells when the subject is a human.

Generally, the methods according to the present invention are suitable for mammals, in particular, for humans.

The subset of B-cells and, in case of humans, human B-cells, may be determined by antibody staining against surface markers. These human B-cells used according to the present invention are B-cells being positive for antibody staining against PD-1; CD19 and/or CD20; and CD80, and being negative for antibody staining against CD14 and/or CD11c, and/or CD66b; CD3 and/or CD4 and/or CD8. Furthermore, the predetermined subset of B-cells is determined based on the immune-tolerance-inducing compound, e.g. being a labelled-tolerance-inducing compound.

In an aspect of the present invention, the method is a method for monitoring ITI in a subject. Further, the method is a prognostic method for accessing ITI success in a subject to undergo ITI treatment. Moreover, the method according to the present invention allows to assist on continuing ITI as further administration strategy by administering the B-cell tolerance inducing compound and to determine the amount, duration or frequency of administration.

In an embodiment, the ITI is an ITI using Factor VIII or Factor IX as immune-tolerance-inducing compound. In particular, the method is a method with ITI using Factor VIII in the treatment of haemophilia A.

In a further aspect, the use of the PD-1 molecule as a marker for the stratification of the therapeutic regimen of a subject afflicted or suspected to be afflicted with an antibody mediated disease based on the B-cell tolerance of said subject is provided. The present inventors recognized that the use of the expression of PD-1 on or by a predetermined set of B-cells, namely the B-cells being specific to the antigen the antibodies of the antibody-mediated disease are directed to, allows to identify the B-cell tolerance status in said subject.

In a further aspect, the present invention relates to the use of the PD-1 molecule for determining development of antibody producing B-cells being specific against an immune-tolerance-inducing compound in a subject treated with said immune-tolerance-inducing compound. In an embodiment, the present invention relates to the use of the PD-1 in determining success of ITI in the treatment of subjects in need thereof, in particular, in subjects afflicted with haemophilia A whereby the ITI is based on administering Factor VIII. Of course, PD-1 can be used as a marker during other treatment courses including administration of compounds by way of replacement therapy. For example, diseases where recombinant molecules or isolated molecules are provided including enzyme replacement therapy, e. g. in case of Fabry disease or Lysosomal storage diseases (Pompe's disease, Gaucher's disease and Fabry's disease).

In a further aspect, the present invention relates to a kit for use in determining antibody producing B-cells by flow cytometry. Said kit comprises suitable antibodies, in particular, optionally labelled with a fluorescence marker whereby these antibodies are preferably monoclonal antibodies. E. g. these antibodies are directed against CD19 and or CD20, PD-1, CD80, CD14 and/or CD11c and/or CD66b, CD3 and/or CD4 and/or CD8. Further, the kit comprises immune-tolerance-inducing compound optionally directly or indirectly labelled or, alternatively, comprises the immune-tolerance-inducing compound and an antibody specifically binding said compound at an epitope different to an epitope recognized by the B-cells. In an embodiment, the kit comprises further an antigen for controlling the efficiency and efficacy of the immune tolerance induction. The kit is for use in determining in a sample of a subject antibody producing B-cells against the allergenic compound or immune-tolerance-inducing compound administered to said subject. The kit for use according to the present invention is particularly useful in a method according to the present invention, e. g. wherein the disease is haemophilia A comprising an antibody against Factor VIII for determining the level or amount of expression of Factor VIII autoreactive B-cells and, consequently, suitable for use as a prognostic marker for ITI success, to assist in the decision whether an ITI should be continued or not, or in case of access, to plan tapering the Factor VIII dose. In addition, the kit is also for use in giving a risk assessment to monitor inhibitor development, whereby switching to a different therapy should be considered. Said kit is also for use in determining in advance whether ITI may be successful or not.

Finally, the present invention relates to a method for ITI treatment comprising, determining the level or amount of PD-1 expression on B-cells involved in immune tolerance induction, namely, expressing specific antibodies directed against the immune-tolerance-inducing compound. For example, in case of ITI treatment in haemophilia A, the immune-tolerance-inducing compound is Factor VIII, either isolated or recombinant.

Based on the PD-1 expression of the Factor VIII specific B-cells, the therapy's regimen is determined. E. g. on case of low amount or level of PD-1, in particular, in case of no increase of the amount or level of expression of PD-1, the therapy, e.g. the ITI should be discontinoued or should be changed by adaption of dosage or interruption for a time. In case of increasing amount or level of expression of PD-1 or in case of normal or high amount or level of expression of PD-1, the therapy should be continued. Further, monitoring the presence of immune tolerance against the specific compound is possible on the basis of the level or amount of expression of PD-1. That is, PD-1 is a suitable marker molecule of the maintenance of immune tolerance induced before.

The specific amount or level of expression of PD-1 on the predetermined B-cells is typically determined individually. For example, the level or amount is determined at the beginning of therapy and monitored thereafter. Alternatively, the level or amount of expression of PD-1 on the alloantigen specific cells is compared to the respective level or amount with alloantigen unspecific B cells present.

In particular in case of hemophilia A, the PD-1 expression level or amount of B-cells identifying factor VIII is determined. The same is true for other diseases including Fabry disease, etc.

Further, the present invention relates to method of treatment subjects afflicted with an immune antibody-mediated disease, including diseases described herein comprising the step of administering to a subject being afflicted with an immune antibody mediated disease, like subjects undergoing ITI, a therapeutically effective amount of an agonist of PD-1, like recombinant PD-L1 are an agonistic antibody against the PD-1 molecule expressed on B-cells. In case of ITI, administration of the agonist of PD-1 may be conducted simultaneously, separately or sequentially with the administration of the immune-tolerance-inducing compound.

In addition the treatment may comprise administration of Tregs expressing PD-L1, thus, suppressing the immune tolerance inducing compound specific B-cells.

The present invention will be described further by way of examples without limiting the same thereto.

### Methods

### Subjects

Human blood samples were obtained from 31 patients with Hemophilia A and 11 healthy subjects. Twenty-nine of the hemophilia patients suffered from a severe phenotype characterized by residual FVIII activities (FVIII:C) below 1%. Two patients were moderately affected (FVIII:C between 1% and 5%). Eleven patients had developed high titer (5 BU or higher) of neutralizing antibodies against FVIII and another 5 patients had a history of low titer (below 5 BU) of neutralizing inhibitors measured by the Nijmegen Bethesda assay. Fifteen patients had not developed neutralizing antibodies to FVIII until blood was drawn for the analysis. Fourteen of them had been exposed to FVIII extensively, however one patient did not receive FVIII before the blood sample was drawn (drug naive control). Of the 16 patients with a history of inhibitors 12 had completed ITI treatment successfully and 2 patients had completed ITI with partial success. One patient had failed in a first ITI attempt and had started a second ITI treatment cycle when blood was drawn. For one adult patient with severe hemophilia A and a long history of high titer neutralizing antibodies to FVIII, ITI treatment was monitored longitudinal. ITI was performed according to a modified Malmö protocol (high dose FVIII, immune suppression with MMF and steroids, i. v. IgG), Freiburghaus, C. et al., Haemoph. Off. J. World Fed. Hemoph. 5, 32-39 (1999). For this patient samples were obtained before start of the first ITI treatment cycle at day -6 and during this first ITI cycle (day 0, 5, 14, and 42). After the first ITI cycle had to be stopped at day 49 further samples were drawn at day 80 and day 114. A second ITI cycle was started at day 154 and blood was obtained at day 155, 159, 171, and 190 (corresponds to day 1, 5, 17, 36 of 2nd ITI cycle). This second ITI cycle was stopped at day 194. Follow-up post ITI samples were taken on day 232 and day 295 (day 78 and 141 of 2nd ITI cycle).

### Human blood samples

PBMCs were obtained from human blood samples by centrifugation in a gradient of Percoll (PromoCell (Merck, Deutschland)). Cells were collected from the gradient and stained as described in "flow cytometry and cell sorting".

### Mice

All mice (C57BL/6, B6;129S-F8tm1Kaz, B7H1-/-, LuciDTR) were bred and maintained under specific pathogen-free conditions at the central animal facility of the University clinic of Bonn (House of Experimental Therapy). Female and male mice were aged 8-14 weeks at the beginning of the experiments. All studies were carried out in accordance with the German animal experimentation law and proven by the relevant local authorities.

### Treatment with FVIII

At weekly intervals mice received 4 intravenous injections of 2IU (0.5 µg or 80IU/kg) recombinant human FVIII protein (rhFVIII, Kogenate^{®}, Bayer Deutschland) diluted in phosphate-buffered saline (PBS; Gibco). For ITI mice received the same doses of FVIII twice a week. The injection sites were physically closed by electric cauterization.

### Immunization with ovalbumin

Mice were immunized with 4 intraperitoneal doses of 10 µg ovalbumin (OVA, Merck Deutschland). The OVA was diluted in PBS and mixed with aluminum hydroxide in a 1:1 ratio in 200 µL of total volume.

### Reagents

CD25+ cells were depleted by injecting 250 µg of PC61.5 antibody (BioXCell (Hölzel) Deutschland)
intraperitoneally one day prior each treatment with FVIII. Foxp3+ cells in LuciDTR mice were depleted by injecting 15 ng/g mouse diphtheria toxin (DTX, Merck Deutschland) interperitoneally at day -1 and 0 of the respective experiment. For blockage of PD-1, 250 µg of the inhibitory antibody RMP1-14 (BioXCell (Hölzel Deutschland) was injected interperitoneally twice a week.

### Blood collection

Blood samples for the evaluation of active FVIII and anti-FVIII antibody titers were obtained by cardiac puncture. For normal blood serum the samples were kept at RT for 30 min. The clotted blood was removed, and the samples were centrifuged for 7 min with 13200 r.p.m. at RT. The serum was transferred into a fresh tube.

For evaluating active FVIII freshly harvested blood was diluted 9:1 with 0.1 mol/L sodium citrate (Merck Deutschland), kept on ice and centrifuged for 20 min with 300xg at RT. The serum was transferred into a fresh tube. The serum samples were subsequently stored at 20°C.

### Active FVIII measurement

Active FVIII in the sodium citrate-buffered serum of mice was determined using COATEST^{®}SP4 FVIII-82 4094 63 (Chromogenix Österreich), following the manufacture's instruction. Coagulation reference was used from Technoclone.

### ELISA

Total anti-FVIII antibody titers in the blood serum of FVIII-treated mice were measures by ELISA. High binding microplates (Greiner bio-one Deutschland) were coated with 1,25 µg/mL rhFVIII diluted in coating buffer (50mM NaHCO3 in PBS, Carl Roth Deutschland) at 4°C overnight. Non-specific binding sites were blocked for 1h at RT using a 1% bovine serum albumin (BSA, Merck Deutschland) in PBS. Starting with a 1:20 dilution, plates were subsequently incubated with a serial dilution of serum samples at 4°C overnight. To bind FVIII-specific IgG antibodies, bound to the immobilized human FVIII protein, the plates were incubated with Biotin-SP conjugated AffiniPure Goat Anti-Mouse IgG (Jackson Immuno Research Großbritannien) in a 1:10000 dilution for 2 h at RT. Subsequently the plates were incubated with streptavidin-labeled horseradish peroxidase (Natutec Deutschland) in a dilution of 1:5000 for 1 h at RT and omega-phenylene diamine dihydrochloride (Thermo Fisher Scientific Deutschland) and H2O2 (Carl Roth Deutschland) were used as substrate to finally detect immobilized anti-FVIII antibodies. The reaction was stopped using 1M H2SO4 (Carl Roth Deutschland).

### ELISpot

For evaluating the number of anti FVIII antibody secreting cells, single cell suspensions of splenocytes were prepared as described in "preparation of spleen cells" and resuspended in X-Vivo medium (Lonza Schweiz) supplemented with 10% FCS, 100U/mL penicillin, 100U/mL streptomycin, and 5.5x10-5 M β-mercaptoethanol (Merck Millipore Deutschland). ELISPOT MultiScreen^{®} plates (Merck Millipore Deutschland) were coated with 1,25 µg/mL rhFVIII diluted in coating buffer (50mM NaHCO3 in PBS, Carl Roth Deutschland) at 4°C overnight. Cells were plated on the plate in a serial dilution starting with 2x106 cells and incubated for 4h at 37°C and 5% CO2. To detect cells antigen-specific B cells secreting anti-FVIII antibodies that have bound to the membrane, the plates were incubated with Biotin-SP conjugated AffiniPure Goat Anti-Mouse IgG (Jackson Immuno Research Großbritannien) in a 1:10000 dilution for 2 h at RT. Subsequently the plates were incubated with streptavidin-labeled horseradish peroxidase (Natutec Deutschland) in a dilution of 1:5000 for 1 h at RT. For the visualization of the antibody spots 3-Amino-9-ethylcarbazole (Merck Deutschland) diluted in 0.1M sodium acetate were used as substrate.

### Preparation of spleen cells

Spleens were obtained at day 22, one day after the last dose of FVIII or OVA and were passed through a 100 µm nylon cell strainer (Greiner bio-one Deutschland) and collected in PBS. Subsequently, the cells were centrifuged for 5 min at 1500 r.p.m at 4°C and cleared from red blood cells by hemolysis using a hypotonic red cell lysis buffer (146mM NH4CI, 10mM NaHCO3 and 2 mM ETDA). Hemolysis was stopped with RPMI 1640 (Life Technologies USA) media supplemented with 2% fetal calf serum (FCS, Thermo Fisher Scientific USA). After centrifugation the cells were finally resuspended in the medium required for the respective analysis.

### Restimulation of splenocytes with recombinant FVIII

Splenocytes were isolated as described as described in "preparation of spleen cells" and resuspended in X-Vivo medium (Lonza Schweiz) supplemented with 10% FCS, 100U/mL penicillin, 100U/mL streptomycin, and 5.5x10-5 M β-mercaptoethanol (Merck Deutschland). Approximately 1x107 splenocytes/well were plated on a 96-well (TPP^{®}) plate and restimulated with 2,5 µg rhFVIII at 37°C and 5% CO2 overnight.

### Isolation and adoptive transfer of primary Tregs

Single cell suspensions from spleens were generated as previously described. CD4+ CD25+ regulatory T cells were further purified using the murine CD4 CD25 Regulatory T Cell Isolation Kit from Miltenyi Biotec (Deutschland )following the manufacturer's instruction. 1x106 cells in PBS were adoptively transferred into acceptor mice by intravenous injections.

### Flow cytometry and cell sorting

Murine splenocytes and human blood cells were centrifuged and stained in PBS supplemented with 0.1% BSA and 0.1% sodium azide (Carl Roth Deutschland) for 30 min at 4°C using fluorochrome-labeled monoclonal antibodies from BioLegend (USA) if not otherwise specified. Unspecific binding sites were blocked with Fc Block (Grifols). Dead cells were excluded using 7-AAD (Thermo Fisher Scientific USA). To identify FVIII-specific B cells, soluble rhFVIII was conjugated to Alexa647 fluorochrome with a commercial kit (Invitrogen USA) and used in a 1:400 dilution. Apoptosis was determined using the Annexin V-FITC Apoptosis Detection Kit (eBioscience USA), together with Hoechst (Molecular Probes USA), following the manufacturer's instruction. For intracellular staining, cells were fixed and stained using the Foxp3/Transcription Factor Staining Buffer Set (eBioscience USA) and following the manufacturer's instruction. To determine absolute cell numbers, 1x105 CaliBRITE APC beads were added before flow cytometry as internal control. Samples were analyzed using a FACSCanto II (BD Biosience USA) or the LSRFortessa (BD Bioscience USA). Cell sorting was performed using the FACSAriaTMIII (BD Biosience USA). Results were analyzed with FlowJo software (FlowJo10.5.3).

The following specific antibodies were used for the staining of murine cells: B220 (RA3-6B2), PD-1 (J43, eBioscience USA), Fas (15A7, eBioscience USA), CD80 (3H5, BD), CD86 (GL-1), CD4 (GK1.5), Foxp3 (150D), CD25 (PC61) , Neuropilin-1 (3E12), Helios (22F6)

The following specific antibodies were used for the staining of human cells: CD19 (HIB19, eBioscience USA), PD-1 (EH12.2H7), CD80 (2D10), CD14 (61D3, eBiosciense USA), CD3 (UCHT1),CD11c (3.9), CD66b (G10F5), CD4 (OKT4), CD8 (RPA-T8), CD20 (2H7).

### RNA extraction from human cells and quantitative PCR

Total RNA was isolated using a RNeasy kit (Qiagen Deutschland) and reverse transcription was performed using the High Capacity RNA-to-cDNA kit (Thermo Fisher USA). Real-time quantitative PCR was performed on LightCycler^{®}480II (Roche Schweiz). The following primers were purchased from Invitrogen and used for quantitative PCR: B7H1 (fwd 5'-TGTACCACGTCTCCCACATAACAG-3 rev, (SEQ. ID. No. 1), 5'-ACCCCACGATGAGGAACAAA-3', SEQ. ID. No. 2), B7DC (fwd 5'-TGACCCTCTGAGTTGGATGGA-3', SEQ. ID. No. 3; rev 5'-GCCGGGATGAAA-GCATGA-3', SEQ. ID. No. 4), PD-1 (fwd 5'-A-GCTTATGTGGGTCCGGC-3' SEQ. ID. No. 5, rev 5'-GGATCCTCAAAGAGGCC-3', SEQ. ID. No. 6), FasL (fwd 5'-CGGTGGTATTTTTCATGGTTCTGG-3', SEQ. ID. No. 7, rev 5'-CTT-GTGGTTTAGGGGCTGGTTGTT-3', SEQ. ID. No. 8), Fas (fwd 5'-TCTGGTGCTT-GCTGGCTCAC-3', SEQ. ID. No. 9; rev 5'-CCATAGGCGATTTCTGGGAC-3', SEQ. ID. No. 10)

### Statistical analysis

We used GraphPad Prism software V8.0.2 for statistical analysis. As specified in the figure legends data are presented as mean +/- SEM. Experiments using mice were performed at least three times (n=3) and a group size between n=4-8 mice. Comparisons were made using ANOVA test with Bonferroni posttest or a paired Student's t-test, depending on the set of data. *P <0.05; **P < 0.01; ***P < 0.001

### Results

### PD-1 restricts the formation of FVIII-inhibiting antibodies in vivo

To identify the molecular mechanism underlying the formation of neutralizing antibodies against transfused FVIII in hemophilia patients, we used transgenic mice with a truncated FVIII protein as a disease model (HemA) have been used (see Velu, V. et al., Nature 458, 206-210 (2009). These mice and control wildtype mice (WT) were injected 4 times in weekly intervals with FVIII or ovalbumin (OVA) as functionally irrelevant control antigen (experimental scheme in Fig. 1a). This protocol induced robust anti-FVIII titers after 22 days in HemA mice injected with FVIII, but not with OVA or in WT control mice injected with FVIII (Fig. 1b), indicating immune-tolerance against FVIII in WT, but not HemA mice. To determine whether such tolerance operated on the B cell level, a flowcytometric staining protocol to identify FVIII-specific B cells was designed. This revealed significantly higher numbers of such B cells in HemA mice injected with FVIII compared to WT mice immunized with that protein, or HemA mice injected with OVA (Fig. 1c), indicating defective deletion of FVIII-specific B cells in HemA mice. This result was confirmed by ELISpot analysis, which also showed more FVIII-specific B cells in these mice. To clarify the underlying molecular mechanism, FVIII-specific B cells for the expression of inhibitory markers were analysed. Remarkably, the percentage of FVIII-specific B cells expressing programmed death receptor 1 (PD-1), and the expression level of this inhibitory molecule on these cells was lower in HemA mice than in WT mice (Fig. 1d, e). Another inhibitory receptor Fas remained unchanged (Fig. 2a. b), whereas activation markers such as CD80 and CD86 were increased on FVIII-specific B cells in HemA mice than in WT controls (Fig. 2c-h). To test the functional relevance of PD-1, WT mice were treated twice a week with an antibody blocking this receptor (RMP1-14) (Fig. 2i, j). Such PD-1 inhibition significantly increased FVIII-specific antibody titers as well as absolute numbers of FVIII-specific B cells to levels comparable to those in HemA mice (Fig. 1f, g). As PD-1 is known to inhibit B cell proliferation, apoptosis of FVIII-specific B cells were analysed. Indeed, more of these B cells were apoptotic in WT than in HemA mice, unless WT mice were treated with PD-1-blocking antibodies (Fig. 1h), verifying that PD-1 was important to establish B cell tolerance against FVIII.

### PD-L1⁺ Tregs are necessary and sufficient to suppress FVIII-specific B cells in vivo

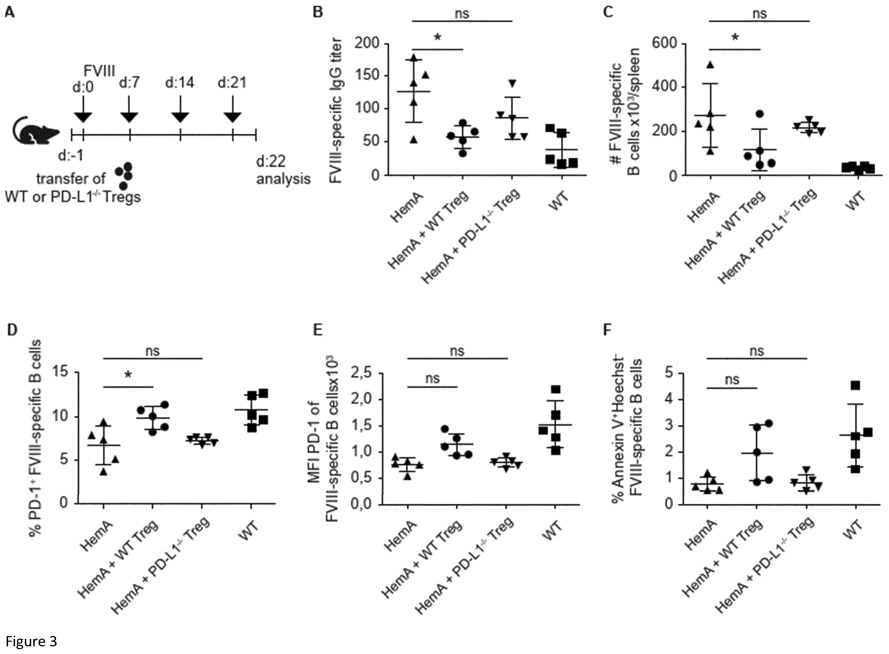

As a next step it had been determined which cell type had established PD-1-dependent B cell tolerance. Regulatory T cells (Tregs), based on their role in maintaining tolerance of autoreactive B cells had been examined. Indeed, more Tregs were found in WT than in HemA mice, and blocking PD-1 reduced these numbers in WT mice to those in HemA mice (Figure 1i). To clarify whether Tregs were necessary for tolerance induction, these cells were depleted with the use of Foxp3-LuciDTR mice (LuciDTR) by injection of diphtheria toxin (DTX) on two consecutive days before injecting FVIII (experimental scheme Fig. 3a). Indeed, Treg depletion enhanced inhibitor titers and FVIII-specific B cell numbers compared to WT controls (Fig. 3b, c). Furthermore, Treg depletion was accompanied by a decrease in PD-1 expression (Fig. 3d, e) and apoptosis maker expression (Fig. 3f) on FVIII-specific B cells. These findings demonstrated that Tregs were necessary for suppression of FVIII-specific B cells *in vivo.* To determine whether Tregs, and PD-L1 expression on them, were sufficient for such tolerance, PD-L1-competent and PD-L1-deficient Tregs were transferred into HemA mice before immunization (experimental scheme Fig. 4a). PD-L1 competent Tregs significantly reduced inhibitor titers and the number of FVIII-specific B cells in HemA mice, whereas PDL1- deficient Tregs were not able to do so (Fig. 4b, c). Furthermore, the transfer of PD-L1+ Tregs elevated PD-1 expression on FVIII-specific B cells as opposed to PD-L1- Tregs (Fig. 4d, e), consistent with a tendency towards higher B cell apoptosis rate after transfer of PDL1+ Tregs (Fig. 4f). These findings demonstrated that PD-L1 expression by Tregs was sufficient for suppression of FVIII-specific B cells *in vivo.*

### Tregs induced via ITI suppress FVIII-specific immunity in a PD-1 dependent manner

The finding that Tregs and the PD-1 axis were important for tolerance to FVIII raised the question whether this mechanism may be involved also in ITI protocols currently being used to prevent inhibitor formation in haemophilia A patients. To answer this question, a treatment protocol that mimics ITI in mice by injecting FVIII twice a week over 21 days was established (experimental scheme Fig. 5a). The protocol increased inhibitor titers at day 21 more than the normal disease treatment scheme consisting of weekly FVIII infusions (Fig. 5b). This is consistent with observations in patients that also showed such an increase after initial antigen exposure, Gouw, S. C. et al., Blood 121, 4046-4055 (2013). Nevertheless, already at this early time point, diminished numbers of FVIII-specific B cells in HemA mice under ITI, but not in mice treated weekly (Fig. 4c) was observed. Furthermore, the percentage of active FVIII after ITI was increased from 0.5 to 2% (Fig. 4d), confirming the effectiveness of our murine ITI protocol. Importantly, enhanced Treg numbers during ITI in comparison to the standard FVIII treatment protocol was observed (Fig. 5e), and these expressed lower levels of Neuropilin-1 and Helios, two markers often used to identify induced regulatory T cells, unless PD-1 was inhibited by an antibody (Fig. 6). Furthermore, the reduced numbers of FVIII specific B cells during ITI (Fig. 5c) correlated with the increased expression of PD-1 by these cells (Fig. 5f, g). These observations suggested a functional role of Tregs and of the PD-1 axis in ITI. To investigate this hypothesis, mice were treated with antibodies blocking PD-1 (RMP1-14) or depleting Tregs (PC61.5). Indeed, both manoeuvres increased the numbers of FVIII-specific B cells (Fig. 5c), reduced PD-1 expression on these cells (Fig. 5f, g) and decreased their expression of apoptosis marker (Fig. 5h). These findings confirmed that the present ITI protocol induced Tregs, which tolerized FVIII-specific B cells in a PD-1-dependent manner.

### Suppression of human FVIII-specific B cells by PD-1 ligation

Finally, it was investigated whether the B cell inhibitory mechanism identified above is also functional in humans. To this end, the fluorescent-FVIII-based flowcytometric staining protocol for FVIII-specific CD19⁺ B cells was adapted to the human system, in order to enable sorting of such B cells from Hemophilia A (HA) patients and healthy volunteers for further analysis. 50 ml of blood were required to obtain enough FVIII-specific B cells for isolating sufficient amounts of mRNA for further analysis (Fig. 7a). The need for such a high volume is a consequence of immune tolerance against self-proteins, which reduces alloreactive B cell frequencies in the blood. FVIII-specific B cells of healthy volunteers, which should be tolerant, expressed higher PD-1 mRNA levels than non-FVIII-specific B cells (Fig. 7b), most of which can be assumed to be specific for foreign antigens. In contrast to PD-1, FVIII-specific B cells expressed neither Fas (Fig. 7c), nor the co-inhibitory molecules PD-L1, PD-L2 or FasL (Fig. 8a-c), suggesting that only PD-1 is operative in tolerant B cells specific for FVIII. A HA patient in the outpatient clinic who developed inhibitors in early childhood and underwent ITI at the age of 40 volunteered to provide that amount of blood at various time points before and during ITI. The patient was suffering from an intron 22 inversion and lacked FVIII secretion. ITI was performed according to a modified Malmö protocol, Zeitler, H. et al., Blood 105, 2287-2293 (2005). In that patient, a large population of FVIII-specific B cells was clearly visible (Fig. 6d). Before the ITI attempt, neither PD-1, nor Fas mRNA expression was detectable in sorted FVIII-specific B cells (Fig. 7b, c), consistent with the inactivity of both signaling pathways in B cells specific for a foreign antigen. Already a few days after ITI, an increase of PD-1 and Fas expression (Fig7e, f), but not of the other co-inhibitory molecules under examination (Fig. 8d-f), was detectable on FVIII-specific B cells. This may be explained by the typical induction of Fas and PD-1 after BCR signaling. In the patient, ITI had to be interrupted after 1 week because of a respiratory infection. When this infection was overcome, another cycle of ITI was initiated 160 days later. Again, a transient PD-1 increase on FVIII-specific B cells was detectable (Fig. 7e).

Importantly, it remained elevated after the actual treatment cycle at least until day 286 (Fig. 7e). Such a second increase was not seen for Fas expression (Fig. 7f). To compare PD-1 expression between that patient and healthy individuals, the ratio of PD-1 expression by FVIII-specific versus non-specific B cells on the RNA level was calculated. During ITI, PD-1 expression increased to levels comparable to healthy donors (Fig. 7g), suggesting a functional role of PD-1 in ITI.

### Discussion

Inhibitory antibodies are an important complication of haemophilia treatment, Kempton, C. L. & Meeks, S. L., Blood 124, 3365-3372 (2014). The immune mechanisms governing inhibitor formation remain incomplete understood. Protocols to induce tolerance against infused coagulation factors are expensive, burdensome and not always effective, and the principles underlying their action are unclear as well. Here we propose a pathophysiological link between inhibitor-producing B cells and Tregs, which is mediated via PD-1/PD-L1 signalling is described. It is suggested that this molecular mechanism is critical for avoiding inhibitor formation in haemophilia A patients and that it underlies ITI therapy, in particular the widely used Bonn protocol. Such ITI is in fact an application of a long known immunological phenomenon termed "high zone tolerance". Tregs are known to suppress autoantibody formation, and have been proposed as therapeutic tool to prevent inhibitor formation. As Tregs are widely accepted to suppress other T cells, it had been assumed that they suppress B cells indirectly by inhibiting fTh cells. There is evidence that Tregs can suppress B cells also directly. Mice expressing transgenic model antigens have been used to demonstrate that PD-1 on autoreactive B cells is critical for such suppression, Gotot, J. et al., Proc. Natl. Acad. Sci. 109, 10468-10473 (2012). This was surprising because fTh cells due to their high PD-1 expression seemed to be the obvious targets of suppression. The present invention demonstrates that inhibitor-producing B cells express PD-1, initially at lower levels, but upregulate it upon encounter with their cognate antigen and that they undergo apoptosis upon direct encounter with PD-L1⁺ Tregs. Such suppression was antigen-specific, likely due to MHC II-restricted antigen presentation by B cells.

The second main finding is that the ITI protocols, like the Bonn ITI protocol operates through PD-1-mediated B cell apoptosis. To formally demonstrate this, a murine model for ITI was established. Its validity is supported by the initial increase of inhibitors early after ITI induction in our model and their subsequently decline, which is also observed in patients under ITI, Brackmann, H. H. & Gormsen, J., Lancet Lond. Engl. 2, 933 (1977). It has been previously suggested that the availability of FVIII as an antigen first drives inhibitor production by specific B cells, but it remained unclear why the titers subsequently decline with a delay of several weeks11. As mentioned the Bonn ITI Protocol has been developed entirely on an empirical basis. It appears that this results from the Treg-mediated induction of B cell apoptosis, stopping inhibitor formation. The inhibitors produced until then will be cleared with time, resulting in the above mentioned delayed inhibitor disappearance. This interpretation is further supported by the increase of active FVIII in mice in the model described herein after 3 weeks, which suggested that no inhibitors were left at this time point to neutralize newly synthesized FVIII. This is consistent with observations in humans under ITI, in which the inhibitor titers declined after about 4-8 weeks. Hence, ITI re-engages the PD-1- mediated tolerance mechanism that operates in HA patients without inhibitors, and pathogenic B cells are removed.

FVIII-specific B cells were detectable in the blood of an adult patient and among the checkpoint molecules known, only their PD-1 expression after ITI therapy followed the expression pattern observed in mouse models. It is demonstrated herein that PD-1 expression on FVIII-specific B cells of patients that did not develop inhibitors or after successful ITI, and of course in healthy control individuals, but not in B cells of a very young patient before ITI. In such patients, FVIII-specific B cells are formally specific for a foreign antigen and should express little PD-1 except after FVIII-infusion. ITI will then induce FVIII-specific PD-L1⁺ Tregs, which subsequently can eliminate FVIII-specific B cells that present FVIII-derived peptides on MHC II.

To formally demonstrate that human FVIII-specific B cells can be eliminated by this pathway, cocultures such B cells with PD-1-stimulating antibodies were established and demonstrated that they underwent apoptosis. Previous studies had described PD-1 on human B cells as an regulator of activation. The findings described herein demonstrate that such PD-1 is functional and important in human B cell tolerance. The functional role of PD-1 expression on FVIII-specific B cells allows for monitoring the expensive ITI. Such a diagnostic tool represents a prognostic marker of ITI success, to assist in the decision whether an ITI should be continued or not, or, in case of success, to plan tapering the FVIII dose. It is also a risk assessment tool to monitor inhibitor development, to decide whether switching from clotting factor substitution therapy to alternative treatment, such as FIX-FX bispecific antibodies, Oldenburg, J. et al., N. Engl. J. Med. 377, 809-818 (2017), should be considered, namely determine the therapy regimen of said subject. Future studies may decide whether PD-1 expression on B cells can detect this situation even before functional test can do.

Furthermore, they also may lay the foundation for future therapies of inhibitor-complicated hemophilia, for example by transferring PD-L1⁺ antigen-specific Tregs through CAR technology. Finally, the antigen-specificity for the suppressive mechanism identified here suggest an involvement also in other genetic diseases where the supplementation of recombinant proteins is complicated by inhibitor formation, such as Fabry's disease.

## Claims

1. A method for the stratification of the therapeutic regimen of a subject afflicted or suspected to be afflicted with an immune antibody-mediated disease based on the immune status of said subject including the steps of
a) providing a sample containing B-cells from said subject;
b) determining the level or amount of the expression of PD-1 in a predetermined subset of B-cells present in said subject, said predetermined B-cells reflect the immune status of said subject;
c) determining the therapeutic regimen of said subject based on the level or amount of the expression of PD-1 in the predetermined subset of B-cells reflecting the immune status of said subject with respect to said disease.

2. A method for monitoring in a subject afflicted with an immune antibody-mediated disease the development or progress of a treatment based on an administration to said subject of an immune-tolerance-inducing compound containing antigenic epitopes recognized by the B-cells producing these antibodies during therapy, including the steps of
a) determining the level or amount of the expression of PD-1 in a predetermined subset of B-cells which are B-cells producing antibodies against the immune-tolerance-inducing compound present in a sample obtained from said subject at a first time point and,
b) optionally, determining the level or amount of the expression of PD-1 in a predetermined subset of B-cells which are B-cells producing antibodies against the immune-tolerance-inducing compound present in a sample obtained from said subject at a second time point; and
c) comparing the level or amount of the expression of PD-1 by said B-cells determined in step a) to the level or amount detected in step b) or to a reference value.

3. A method for determining the risk of developing antibody-producing B-cell based failure of immune tolerance induction (ITI) treatment in a subject undergoing said treatment to ITI by administering an immune-tolerance-inducing compound comprising the steps of
a.) determining the level or amount of the expression of PD-1 in a predetermined subset of B-cells present in said sample at a first time point, said B-cells being specific against the immune tolerance inducing compound;
b.) optionally determining the level or amount of the expression of PD-1 in said predetermined subset of B-cells being specific against the immune tolerance inducing compound present in a sample obtained from said subject at a second time point;
c.) determining the risk of failure of ITI therapy in said subject based on the level or amount of the expression of PD-1 in a predetermined subset of B-cells being specific against the immune tolerance inducing compound.

4. The method according to any one of the preceding claims wherein the predetermined subset of B-cells are B-cells which produce antibodies directed against the B-cell tolerance-inducing compound administered in the ITI therapy.

5. The method according to any one of the preceding claims wherein the subject is a subject afflicted with hemophilia A and the predetermined subset of B-cells are antibody-producing B-cells against factor VIII.

6. The method according to any one of the preceding claims wherein the therapeutic regimen or the treatment is an ITI therapy, the disease is hemophilia A and the tolerance inducing compound administered is factor VIII.

7. The method according to any one of the preceding claims wherein determining the level or amount of the expression of PD-1 in a predetermined subset of B-cells is by flow cytometry.

8. The method according to any one of the preceding claims wherein the sample containing B-cells is obtained from blood, in particular, serum.

9. The method according to any one of the preceding claims wherein the predetermined subset of B-cells are human B-cells and which are determined by being positive for antibody staining against PD-1; CD19 and/or CD20; and CD80, and being negative for antibody staining against CD14 and/or CD11c, and/or CD66b; CD3 and/or CD4 and/or CD8.

10. A method according to any one of the preceding claims for monitoring ITI, as a prognostic method for assessing ITI success, to assist on continuing ITI as further administration strategy by administering the immune-tolerance-inducing compound, in particular, Factor XIII in case of the treatment of hemophilia A.

11. The use of PD-1 as a marker for the stratification of the therapeutic regimen of a subject afflicted or suspected be afflicted with an antibody-mediated disease based on the B-cell tolerance status of said subject.

12. The use of PD-1 according to claim 11 for determining the development of antibody-producing B-cells being specific against an immune-tolerance-inducing compound in a subject treated with said immune-tolerance-inducing compound.

13. The use of PD-1 according to claim 11 or 12 in determining success of ITI in the treatment of subjects in need thereof, in particular, in subjects afflicted with haemophilia A.

14. A kit for use in determining antibody-producing B-cells by flow cytometry comprising antibodies, said antibodies being optionally labeled with a fluorescence marker, said antibodies are preferably monoclonal antibodies, and being directed against CD19 and/or CD20, PD-1, CD80, CD14 and/or CD11c and/or CD66b, CD3 and/or CD4 and/or CD8, and the fluorescenctly labelled allogenic compound administered to a subject.

15. The kit according to claim 14 for use in a method according to any one of claims 1 to 10 wherein the disease is hemophilia A comprising an antibody against factor VIII for determining the level or amount of factor VIII auto reactive B-cells.
